# EUROPEAN PATENT SPECIFICATION

(11) **EP 4 005 530 B1**
(45) Date of publication and mention of the grant of the patent: **25.12.2024**
(21) Application number: 21209348.8
(22) Date of filing: 19.11.2021
(51) Int. Cl.: A61C 19/00, A61J 7/00

(54) **BONE GRAFT CARRIER**
TRÄGER FÜR KNOCHENTRANSPLANTAT
SUPPORT POUR GREFFE OSSEUSE

(30) Priority: 26.11.2020 TW 109141545
(43) Date of publication of application: 01.06.2022
(73) Proprietor: Shih, Jui-Yuan, Lukang Township, Changhua County (TW)
(72) Inventor: Shih, Jui-Yuan, Lukang Township, Changhua County (TW)
(74) Representative: Melchior, Robin

(56) References cited:
- CN-Y- 201 266 149
- GB-A- 2 159 129
- KR-A- 20120 066 212
- KR-B1- 101 325 898
- US-A- 4 800 875
- US-A- 636 735
- US-A- 884 390

## Description

### BACKGROUND

### 1. Field of the Invention

The present invention relates to a bone graft carrier that can be used conveniently, reduces the cost, and can be operated accurately.

### 2. Description of Related Art

With the advancement and development of science and technology, the current technology and equipment related to dental health and treatment are also flourishing. Among them, the dental implants have gradually become one of the indispensable life necessities of the elderly people. In the process of inserting artificial dental implant, different amounts of bone graft will be filled into the patient's alveolar bone according to the atrophic conditions of the patient's alveolar bone, so as to repair, strengthen, and arrange the patient's alveolar bone to ensure that the artificial dental implant is implanted into the patient's alveolar bone and is securely disposed in the patient's alveolar ridge.

Furthermore, the types of bone graft can be roughly divided into artificial bone graft, autologous bone graft, allogeneic bone graft, xenograft, etc. The artificial bone graft is made of synthetic chemical materials. The autologous bone graft is obtained from the patient's own bones such as cranium, mandibular ramus, patella, etc. The allogeneic bone graft is made from human bones of the donor. The xenograft uses animal bones such as pig bones and cattle bones. Each of the above-mentioned types of bone graft has its advantages and disadvantages. However, the cost is generally more expensive. A few grams of bone graft costs NT 1000 to 10,000, which is quite a financial burden to patients.

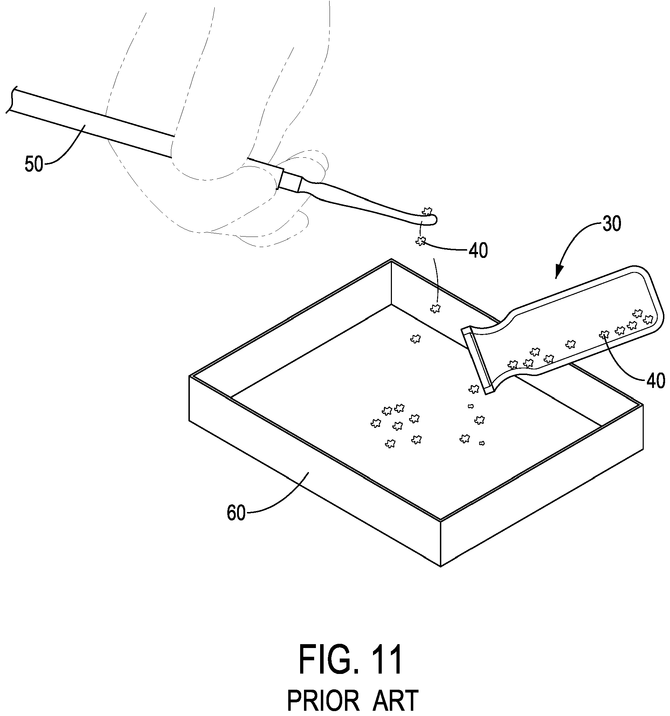

Additionally, during the dental implant process, when bone graft is needed for filling, as shown in Fig. 11, bone graft 40 stored in a bottle 30 is poured into a bowl 60 with an upper opening. The bowl 60 is placed on a side of the patient undergoing a dental implant surgery, and the dentist will use a periosteal elevator 50 (PE) to dig out the bone graft 40 in the bowl 60, move the bone graft 40 in the periosteal elevator 50 to the patient's mouth, and fill the bone graft 40 into the patient's alveolar bone to repair or strengthen the gum structure.

However, during the movement of transferring the bone graft 40 from the bowl 60 to the patient's mouth, the bone graft 40 is easy to fall from the periosteal elevator 50, it may waste the bone graft 40 and increase the time required to repeatedly pick the bone graft 40 during the dental implant process. When the bone graft 40 is filled in the patient's alveolar bone from the periosteal elevator 50, part of the bone graft 40 will also fall in the patient's mouth from the periosteal elevator 50, and the patient will feel that foreign bodies have entered the mouth, which affects the quality of the operation and causes discomfort to the patient. Therefore, the conventional method and related bowl 60 or container have problems such as inconvenience of use, being unable to effectively save the cost of using the bone graft 40, and being unable to accurately fill the bone graft 40 into the patient's alveolar bone. Therefore, the conventional method and related devices for bone graft procedure for dental implants need further improvement.

In addition, another conventional method and related devices for bone graft are also disclosed in KR 101325898B and US 4 800 875 A, also medical spoons or funnels are disclosed in CN 201 266 149 Y, and US 884 390 A.

### SUMMARY OF THE INVENTION

To overcome the shortcomings, the present invention tends to provide a bone graft carrier to mitigate or obviate the aforementioned problem.

The main objective of the invention is to provide a bone graft carrier that can be used conveniently, reduces the cost, and can be operated accurately.

The bone graft carrier of the invention is defined in claim 1.

The bone graft carrier in accordance with the present invention has a body. The body has a containing portion, a conveying portion, an opening, and an annular flange. The containing portion is formed at one of two ends of the body and has a chamber. The conveying portion is formed at the other one of the two ends of the body, is tapered, is connected to the containing portion, and has a delivery channel communicating with the chamber of the containing portion. The opening is formed through a top surface of the body and directly communicates with the chamber of the containing portion and the delivery channel of the conveying portion. The annular flange is formed on and protruded outwardly from the top surface of the body along an outer periphery of the opening for abutting two fingers when holding the bone graft carrier.

Other objects, advantages and novel features of the invention will become more apparent from the following detailed description when taken in conjunction with the accompanying drawings.

### BRIEF DESCRIPTION OF THE DRAWINGS

Fig. 1 is a perspective view of a first embodiment of a bone graft carrier in accordance with the present invention;
Fig. 2 is a side view of the bone graft carrier in Fig. 1;
Fig. 3 is a cross sectional side view of the bone graft carrier in Fig. 1;
Fig. 4 is an operational perspective view of the bone graft carrier in Fig. 1;
Fig. 5 is another operational perspective view of the bone graft carrier in Fig. 1;
Fig. 6 is a further operational perspective view of the bone graft carrier in Fig. 1;
Fig. 7 is a top side view of a second embodiment of a bone graft carrier in accordance with the present invention;
Fig. 8 is a top side view of a third embodiment of a bone graft carrier in accordance with the present invention;
Fig. 9 is a side view of a fourth embodiment of a bone graft carrier in accordance with the present invention;
Fig. 10 is a side view of a fifth embodiment of a bone graft carrier in accordance with the present invention; and
Fig. 11 is an operational perspective view of a bone graft filling surgery in accordance with the prior art.

### DETAILED DESCRIPTION OF THE INVENTION

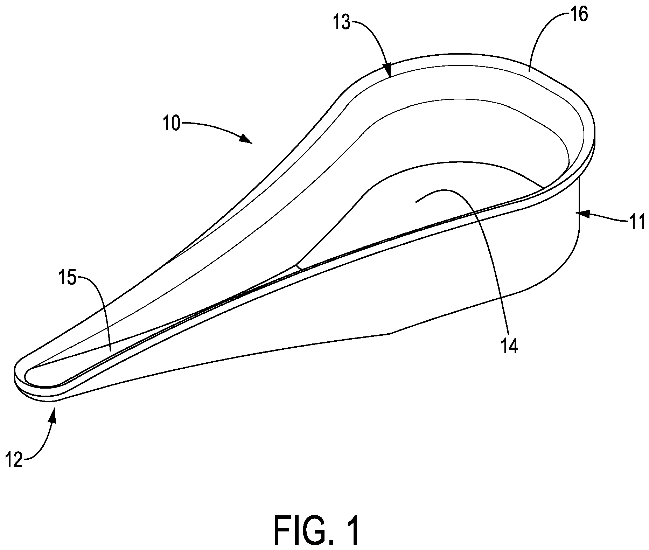

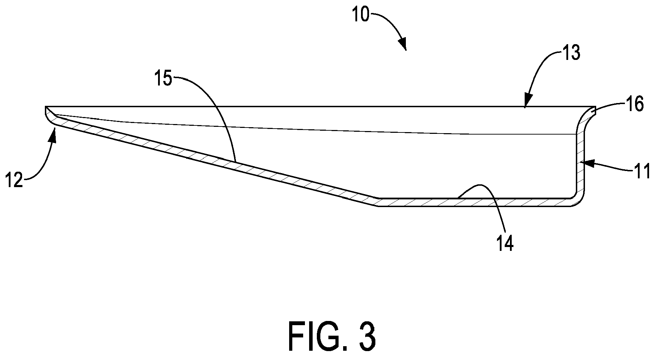

With reference to Figs. 1 to 3, a first embodiment of a bone graft carrier in accordance with the present invention comprises a body 10, and the body 10 may be a container in a drop shape and has two ends, a top surface, a containing portion 11, a conveying portion 12, and an opening 13. The containing portion 11 is formed at one of the two ends of the body 10, may be arc-shaped, and has a chamber 14. The conveying portion 12 is formed at the other one of the two ends of the body 10, is tapered, and is connected to the containing portion 11. The conveying portion 12 has a delivery channel 15 communicating with the chamber 14 of the containing portion 11.

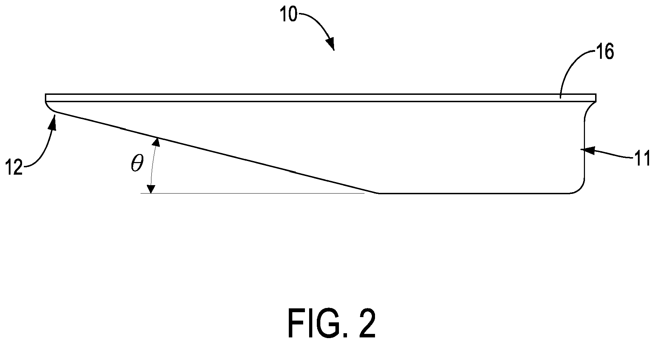

Preferably, the conveying portion 12 extends obliquely upward from a bottom surface of the containing portion 11, and the top surface of the body 10 is horizontal. With reference to Fig. 2, an included angle Θ is formed between a bottom surface of the conveying portion 12 and the bottom surface of the containing portion 11. The included angle Θ is larger than or equal to 0 degree and is smaller than or equal to 90 degrees (0° ≦ Θ ≦ 90°). The opening 13 is formed through the top surface of the body 10 and directly communicates with the chamber 14 and the delivery channel 15. According to the invention, the body 10 has an annular flange 16 formed on and protruded outwardly from the top surface of the body 10 along an outer periphery of the opening 13 for abutting two fingers when holding the bone graft carrier.

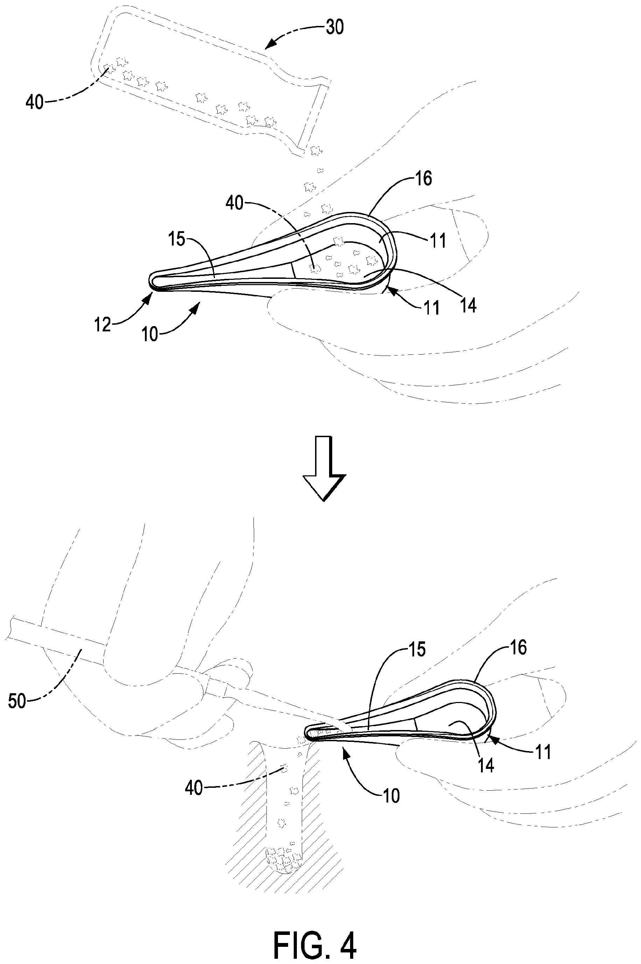

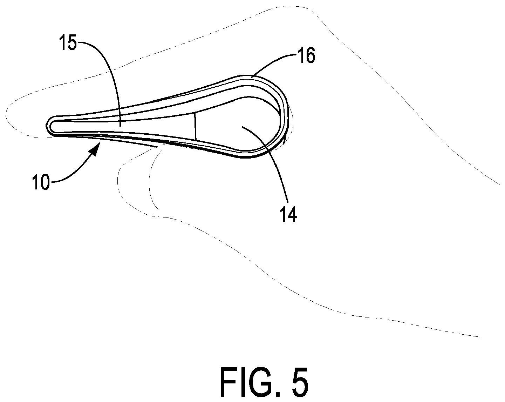

With reference to Fig. 4, when the first embodiment of the bone graft carrier of the present invention is in use, bone graft 40 stored in a bottle 30 is poured into the chamber 14 of the containing portion 11 via the opening 13, and the dentist holds the containing portion 11 by hand and moves the bone graft 40 in the chamber 14 toward a patient's mouth. Further, the dentist can move the body 10 by holding the containing portion 11 with two fingers and abutting the annular flange 16 as shown in Fig. 4 or by covering the body 10 with two fingers and abutting the annular flange 16 as shown in Fig. 5. Then the bone graft 40 in the chamber 14 can be moved to the patient's mouth stably without falling into the surrounding environment. In addition, clinically, as shown in Fig. 4, after a dental drill drills the patient's alveolar bone, the patient's autologous bone graft attached to the dental drill can be removed and collected in the chamber 14 of the containing portion 11 or the delivery channel 15 of the conveying portion 12 via the opening 13 to fully use the patient's autologous bone graft.

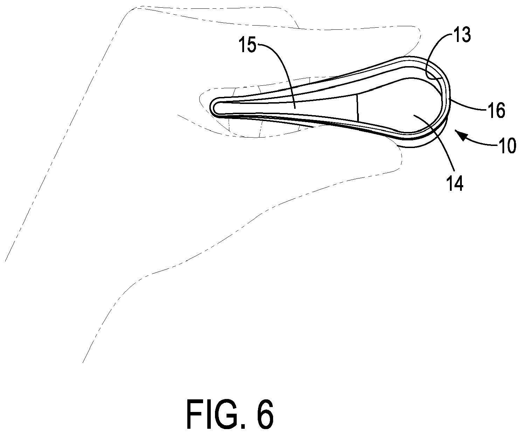

When the body 10 moves to the patient's mouth, the tapered conveying portion 12 is moved to extend to the patient's alveolar bone where the bone graft 40 needs to be filled. At this time, the dentist uses a periosteal elevator 50 extending into the chamber 14 to push the bone graft 40 toward the delivery channel 15 so that the bone graft 40 can pass through the delivery channel 15 and the opening 13 to enter and fill the alveolar bone of the patient, thereby facilitating the filling operation of the bone graft 40. Furthermore, the dentist may replenish the amount of the bone graft 40 according to the patient's needs. With reference to Fig. 6, when a large amount of the bone graft 40 needs to be replenished, the dentist can cover the body 10 with two fingers to transport the bone graft 40 from the chamber 14 of the containing portion 11.

According to the above-mentioned structural relationship and features of the first embodiment of a bone graft carrier in accordance with the present invention, in use, it is only necessary to pour the bone graft 40 into the body 10 and move the body 10 to the patient's mouth, and then the bone graft 40 can be transported to the patient's alveolar bone to be filled through the body 10 to perform the filling operation of the bone graft 40. With the bone graft carrier of the present invention, the dentist does not have to use the periosteal elevator 50 to dig up the bone graft 40 and move the bone graft 40 to the patient's mouth, preventing falling and wasting of the bone graft 40, and there is no need to repeatedly take the bone graft 40, which can greatly shorten the time required to fill the bone graft 40.

In addition, the transportation of the bone graft 40 through the delivery channel 15 of the conveying portion 12 can effectively prevent the bone graft 40 from falling into the patient's mouth, greatly improving the quality of the operation without causing discomfort to the patient, so that the dentist can use it conveniently and accurately, while reducing the burden on the patient to pay for the bone graft 40, and allowing the patient to undergo the operation in a comfortable condition. With reference to Figs. 5 and 6, the dentist can transport the bone graft 40 via the delivery channel 15 of the conveying portion 12 or the chamber 14 of the containing portion 11 according to the actual situation, thereby providing a convenient, cost-saving and accurate operation of the bone graft carrier.

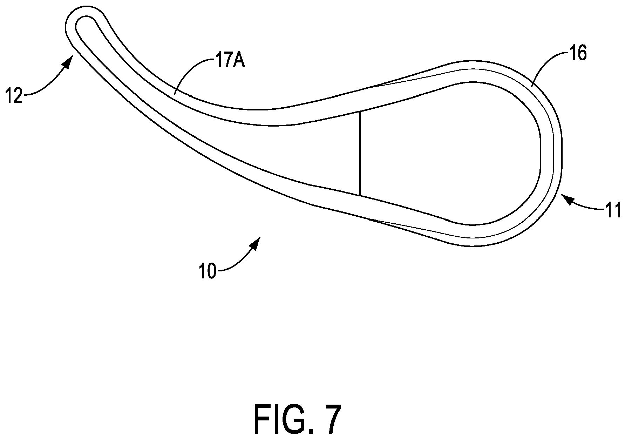

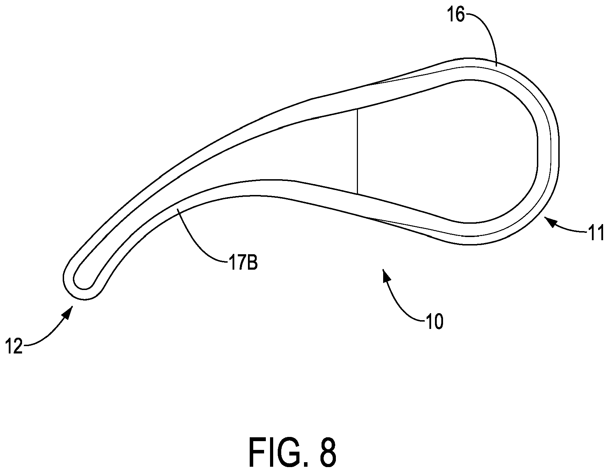

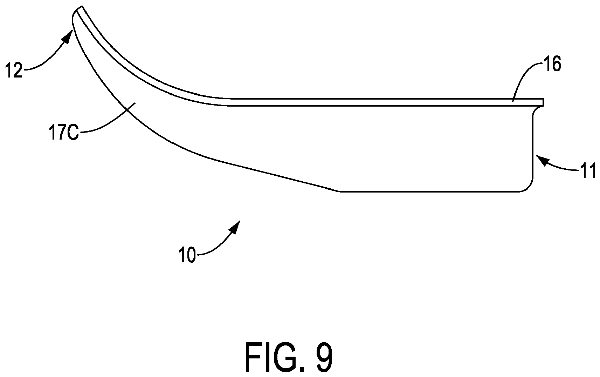

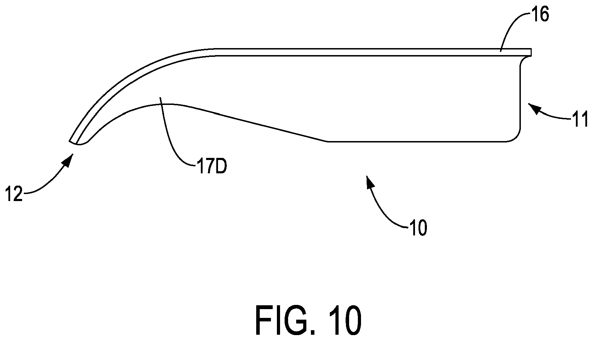

With reference to Figs. 7 to 10, a second, a third, a fourth, and a fifth embodiment in accordance with the present invention are all substantially the same as the first embodiment except for the following features. In the above-mentioned second to fifth embodiments, in order to fill the bone graft 40 to the patent's alveolar bone at different positions, the body 10 has a curved portion formed between the containing portion 11 and the conveying portion 12, and a curved angle is formed between the conveying portion 12 and the containing portion 11. With reference to Fig. 7, in the second embodiment of the present invention, the conveying portion 12 is deflected to a right side of the containing portion 11 via the curved portion 17A. With reference to Fig. 8, in the third embodiment of the present invention, the conveying portion 12 is deflected to a left side of the containing portion 11 via the curved portion 17B. With reference to Fig. 9, in the fourth embodiment of the present invention, the conveying portion 12 is bent upwardly relative to the containing portion 11 via the curved portion 17C. With reference to Fig. 10, in the fifth embodiment of the present invention, the conveying portion 12 is bent downwardly relative to the containing portion 11 via the curved portion 17D.

When the second to fifth embodiments of the bone graft carrier of the present invention are in use, the operations of the above-mentioned embodiments are the same as that of the first embodiment, and it allows the dentist to use the bone graft carrier with the curved portions 17A, 17B, 17C, and 17D according to the positions of the patient's alveolar bone that needs filling of the bone graft 40. The dentist can perform the filling operations of the bone graft 40 more smoothly and closer to the position of the patient's alveolar bone that needs to be filled with the bone graft 40.

## Claims

1. A bone graft carrier, wherein the bone graft carrier comprises a body (10), and the body (10) comprising:
two ends;
a top surface;
a containing portion (11) formed at one of the two ends of the body (10) and having a chamber (14);
a conveying portion (12) formed at the other one of the two ends of the body (10), being tapered, connected to the containing portion (11), and having a delivery channel (15) communicating with the chamber (14) of the containing portion (11); and
an opening (13) formed through the top surface of the body (10) and directly communicating with the chamber (14) of the containing portion (11) and the delivery channel (15) of the conveying portion (12);
**characterized in that**
the body (10) has an annular flange (16) formed on and protruded outwardly from the top surface of the body (10) along an outer periphery of the opening (13) for abutting two fingers when holding the bone graft carrier.

2. The bone graft carrier as claimed in claim 1, wherein the conveying portion (12) extends obliquely upward from a bottom surface of the containing portion (11), and the top surface of the body (10) is horizontal.

3. The bone graft carrier as claimed in claim 1, wherein an included angle (Θ) is formed between a bottom surface of the conveying portion (12) and a bottom surface of the containing portion (11).

4. The bone graft carrier as claimed in claim 3, wherein the included angle (Θ) is larger than or equal to 0 degree and is smaller than or equal to 90 degrees.

5. The bone graft carrier as claimed in claim 1, wherein the containing portion (11) is arc-shaped.

6. The bone graft carrier as claimed in any one of claims 1 to 5, wherein the body (10) is a container in a drop shape.

7. The bone graft carrier as claimed in claim 1 or 2, wherein an included angle (Θ) is formed between a bottom surface of the conveying portion (12) and a bottom surface of the containing portion (11).

8. The bone graft carrier as claimed in claim 7, wherein the included angle (Θ) is larger than or equal to 0 degree and is smaller than or equal to 90 degrees.

## Patentansprüche

1. Träger für Knochentransplantat, wobei der Träger für Knochentransplantat einen Körper (10) umfasst und der Körper (10) Folgendes umfasst:
zwei Enden;
eine Deckfläche;
einen Aufnahmeabschnitt (11), der an einem der zwei Enden des Körpers (10) ausgebildet ist und eine Kammer (14) aufweist;
einen Weiterleitungsabschnitt (12), der an dem anderen der zwei Enden des Körpers (10) ausgebildet ist, verjüngt ist, mit dem Aufnahmeabschnitt (11) verbunden ist und einen Zuführkanal (15) aufweist, der mit der Kammer (14) des Aufnahmeabschnitts (11) in Verbindung steht; und
eine Öffnung (13), die durch die Deckfläche des Körpers (10) ausgeformt ist und direkt mit der Kammer (14) des Aufnahmeabschnitts (11) und dem Zuführkanal (15) des Weiterleitungsabschnitts (12) in Verbindung steht;
**dadurch gekennzeichnet, dass**
der Körper (10) einen ringförmigen Flansch (16) aufweist, der auf der Deckfläche des Körpers (10) und nach außen von ihr vorstehend entlang eines Außenumfangs der Öffnung (13) zum Anlegen von zwei Fingern beim Halten des Trägers für Knochentransplantat ausgebildet ist.

2. Träger für Knochentransplantat nach Anspruch 1, wobei sich der Weiterleitungsabschnitt (12) von einer Bodenfläche des Aufnahmeabschnitts (11) schräg nach oben erstreckt und die Deckfläche des Körpers (10) horizontal ist.

3. Träger für Knochentransplantat nach Anspruch 1, wobei ein eingeschlossener Winkel (Θ) zwischen einer Bodenfläche des Weiterleitungsabschnitts (12) und einer Bodenfläche des Aufnahmeabschnitts (11) gebildet ist.

4. Träger für Knochentransplantat nach Anspruch 3, wobei der eingeschlossene Winkel (Θ) größer oder gleich 0 Grad ist und kleiner oder gleich 90 Grad ist.

5. Träger für Knochentransplantat nach Anspruch 1, wobei der Aufnahmeabschnitt (11) bogenförmig ist.

6. Träger für Knochentransplantat nach einem der Ansprüche 1 bis 5, wobei der Körper (10) ein tropfenförmiger Behälter ist.

7. Träger für Knochentransplantat nach Anspruch 1 oder 2, wobei ein eingeschlossener Winkel (Θ) zwischen einer Bodenfläche des Weiterleitungsabschnitts (12) und einer Bodenfläche des Aufnahmeabschnitts (11) gebildet ist.

8. Träger für Knochentransplantat nach Anspruch 7, wobei der eingeschlossene Winkel (Θ) größer oder gleich 0 Grad ist und kleiner oder gleich 90 Grad ist.

## Revendications

1. Support pour greffon osseux, dans lequel le support pour greffon osseux comprend un corps (10) et le corps (10) comprenant :
deux extrémités ;
une surface supérieure ;
une partie de confinement (11) formée au niveau de l'une des deux extrémités du corps (10) et ayant une chambre (14) ;
une partie de transport (12) formée au niveau de l'autre des deux extrémités du corps (10), étant progressivement rétrécie, raccordée à la partie de confinement (11) et ayant un canal d'administration (15) communiquant avec la chambre (14) de la partie de confinement (11) ; et
une ouverture (13) formée à travers la surface supérieure du corps (10) et communiquant directement avec la chambre (14) de la partie de confinement (11) et le canal d'administration (15) de la partie de transport (12) ;
**caractérisé en ce que** :
le corps (10) a une bride annulaire (16) formée sur et faisant saillie vers l'extérieur à partir de la surface supérieure du corps (10) le long d'une périphérie externe de l'ouverture (13) pour venir en butée contre deux doigts, lors du maintien du support pour greffon osseux.

2. Support pour greffon osseux selon la revendication 1, dans lequel la partie de transport (12) s'étend de manière oblique vers le haut à partir d'une surface inférieure de la partie de confinement (11), et la surface supérieure du corps (10) est horizontale.

3. Support pour greffon osseux selon la revendication 1, dans lequel un angle inclus (Θ) est formé entre une surface inférieure de la partie de transport (12) et une surface inférieure de la partie de confinement (11).

4. Support pour greffon osseux selon la revendication 3, dans lequel l'angle inclus (Θ) est supérieur ou égal à 0 degré et est inférieur ou égal à 90 degrés.

5. Support pour greffon osseux selon la revendication 1, dans lequel la partie de confinement (11) est en forme d'arc.

6. Support pour greffon osseux selon l'une quelconque des revendications 1 à 5, dans lequel le corps (10) est un contenant en forme de goutte.

7. Support pour greffon osseux selon la revendication 1 ou 2, dans lequel un angle inclus (Θ) est formé entre une surface inférieure de la partie de transport (12) et une surface inférieure de la partie de confinement (11).

8. Support pour greffon osseux selon la revendication 7, dans lequel l'angle inclus (Θ) est supérieur ou égal à 0 degré et est inférieur ou égal à 90 degrés.
